## Europäisches Patentamt
## European Patent Office
## Office européen des brevets

(19)

(11) Veröffentlichungsnummer: **0 252 411**
**A2**

(12) **EUROPÄISCHE PATENTANMELDUNG**

(21) Anmeldenummer: **87109373.8**

(22) Anmeldetag: **30.06.87**

(51) Int. Cl.4: **C07G 11/00 , C12P 1/06 , C12N 1/20 , A61K 35/66 , //(C12P1/06,C12R1:29,C12N1:2-0,C12R1:29)**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 3777.

(30) Priorität: **05.07.86 DE 3622664**

(43) Veröffentlichungstag der Anmeldung: **13.01.88 Patentblatt 88/02**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Nadkarni, Suresh Rudra, Dr.**
**5, Yagneshwar Kasturba Marg Mulund (West)**
**Bombay 400 080(IN)**
Erfinder: **Mukhopadhyay, Triptikumar, Dr.**
**M23/24, Hoechst Quarters Near Jai Shastri Nagar**
**Mulund (West) Bombay 400 082(IN)**
Erfinder: **Desikan, Kalyanapuram Rajagopalan**
**4, Krishna Kunj Opp Johnson, L.B.S. Marg**
**Mulund (West) Bombay 400 080(IN)**
Erfinder: **Rupp, Richard Helmut, Dr.**
**Niladri 66 Nepean Sea Road**
**Bombay 400 006(IN)**
Erfinder: **Ganguli, Bimal Naresh, Dr.**
**Saurayuth 173 Central Avenue**
**Chembur Bombay 400 071(IN)**
Erfinder: **Kraemer, Hans Peter, Dr.**
**Birkenweg 16**
**D-3550 Marburg(DE)**
Erfinder: **Sedlacek, Hans Harald, Dr.**
**Sonnenhang 3**
**D-3550 Marburg(DE)**

(54) **Neue Antibiotika, ein mikrobiologisches Verfahren zu ihrer Herstellung und ihre Verwendung als Arzneimittel.**

(57) Die vorliegende Erfindung betrifft neue antibiotisch wirksame Verbindungen, Hextamycin A und Hextamycin B, sowie ein Verfahren zur Herstellung dieser Verbindungen mit Hilfe des Mikroorganismus Mikromonospora species Y-8.20012 (DSM 3777), dessen Varianten und Mutanten.

Die erfindungsgemäßen Verbindungen zeichnen sich auch durch eine hohe cytostatische Wirksamkeit aus und können daher als Arzneimittel verwendet werden.

EP 0 252 411 A2

Xerox Copy Centre

## Neue Antibiotika, ein mikrobiologisches Verfahren zu ihrer Herstellung und ihre Verwendung als Arzneimittel

Die vorliegende Erfindung betrifft neue antibiotisch wirksame Verbindungen, nachstehend als Hextamycin A und Hextamycin B bezeichnet, sowie ein Verfahren zur Herstellung dieser Verbindungen mit Hilfe des Mikroorganismus Micromonospora species Y-82.20012 (DSM 3777), dessen Varianten und Mutanten.

Micromonospora bilden nachweislich mehrere Arten von Antibiotika wie Aminoglykoside, Makrolide, Ansamycine, Evernomicine und Aktinomycine. Antibiotika aus Micromonospora werden in den Annual Reviews of Microbiology, Bd. 34, Seiten 537-57 (l980) beschrieben.

Die hier beschriebenen Antibiotika scheinen sich von diesen bekannten Arten von Antibiotika in ihren biologischen und chemischen Eigenschaften zu unterscheiden und sind aus diesem Grunde Gegenstand der Erfindung.

Die Mikroorganismen für die Herstellung der Hextamycine A und B gehören zu der Ordnung Actinomycetales, Familie Micromonosporaceae und dem Genus Micromonospora.

Der Mikroorganismus Micromonospora species Y-82.20012 wurde am 20.6.l986 unter der Nummer DSM 3777 bei der Deutsche Sammlung vom Mikroorganismen (Griesebachstr. 8, D-3400 Göttingen) hinterlegt.

Das Verfahren zur Herstellung von Hextamycin A und Hextamycin B ist dadurch gekennzeichnet, daß der Mikroorganismus Micromonospora species Y-82.20012 (DSM 3777) seine Varianten oder Mutanten auf übliche Weise unter aeroben Bedingungen in einem Nährmedium kultiviert wird, das Kohlenstoff-und Stickstoffquellen, anorganische Nährsalze, Spurenelemente und ggf. Inhibitoren von Antibiotika-abbauenden Enzymen enthält und die gebildeten Verbindungen aus der Kulturbrühe und dem Myzel wie im folgenden beschrieben, isoliert und gereinigt werden.

Kohlenstoff-Lieferanten können Stärke, Glukose, Saccharose, Dextrin, Fruktose, Melassen, Laktose und Galaktose sein. Bevorzugter Kohlenstoff-Lieferant ist Glukose. Stickstoff-Lieferanten können organische Stickstoffquellen wie Sojabohnenmehl, Erdnußmehl, Hefeextrakt, Malzextrakt, Rinderextrakt, Maislauge, Pepton, Kasein oder Kaseinhydrolysat oder anorganische Stickstoffquellen wie Ammoniumsalze, Ammoniumchlorid, Ammoniumsulfat oder Nitratsalze wie Natriumnitrat oder Kaliumnitrat sein. Als organische Stickstofflieferanten werden Sojabohnenmehl, Pepton und Maislauge bevorzugt. Die bevorzugte anorganische Stickstoffquelle ist Ammoniumsulfat. Als organische Nährstoffsalze können Natriumchlorid, Calciumcarbonat, Natrium-oder Kaliumhydrogen-oder -dihydrogenphosphat, Calciumchlorid, Magnesiumphosphat oder Calciumphosphat dienen. Spurenelemente können die Salze von Eisen, Mangan, Kupfer, Zink oder Kobalt oder anderer Schwermetalle sein. Als Inhibitoren von Antibiotika-abbauenden Enzyme können Jod oder dessen Salze wie Kaliumjodid, Natriumjodid oder Natriumperjodat dienen. Bevorzugter Inhibitor ist Kaliumjodid.

Die Kultivierung von Micromonospora Y-82.20012 erfolgt bei Temperaturen zwischen 28°C bis 32°C und einem pH zwischen 6,0 und 8,0. Mikromonospora species Y-82.20012 wird vorzugsweise bei 28°C und pH 6,l kultiviert. Die Kultivierung wird nach 72 bis 96, bevorzugt 72 Stunden beendet, wenn optimale Ausbeuten der Substanzen der vorliegenden Erfindung gewonnen worden sind. Die Kultivierung erfolgt vorzugsweise als Submers-Kultivierung. Zu Beginn der Kultivierung kann ein Antischaum-Mittel, bis zu einer Endkonzentration von 0,025 bis 0,5 % der Kulturbrühe zugesetzt werden. Als Antischaum-Mittel kann Pflanzenöl, z.B. Erdnußöl, Maisöl, Sojabohnenöl oder ein Silikonöl wie beispielsweise [R]Desmophen dienen. Als Antischaum-Mittel wird [R]Desmophen 0,04 % bevorzugt.

Der Verlauf der Kultivierung und die Bildung der Hextamycine gemäß der vorliegenden Erfindung kann mittels Messung der antibakteriellen Wirkung der Kulturbrühe gegen Bakterienstämme wie B. subtilis, S. aureus, E. coli, Ps. aeruginosa und einige andere überwacht werden. Bevorzugt wird dafür Ps. aeruginosa verwendet.

In der entstandenen Kulturbrühe liegen die Hextamycine gemäß dieser Erfindung sowohl im Myzel als auch im Kulturfiltrat vor. Die Hextamycine werden vorzugsweise aus dem Myzel isoliert, das durch Filtrieren oder Zentrifugieren der Kulturbrühe gewonnen wird. Das Myzel wird mit Lösungsmitteln wie Methanol, Aceton, Äthanol, n-Butanol oder Äthylmethylketon extrahiert. Als Lösungsmittel wird Aceton bevorzugt. Der Lösungsmittelextrakt wird unter Vakuum konzentriert und filtriert. Nach der Verdünnung mit Wasser wird das Filtrat auf eine [R]Diaion HP-20-Säule gegeben und iann mit Wasser-Methanol eluiert. Die aktiven Methanoleluate werden konzentriert und dann mit Petroläther (Sp. 60° bis 80°C) behandelt. Die Hextamycine werden aus dem durch Filtrieren oder Zentrifugieren der Kulturbrühe gewonnenen Kulturfiltrat durch Extraktion mit einem mit Wasser nicht mischbaren Lösungsmittel wie Äthylacetat, Chloroform oder Äthylmethylketon gewonnen, nachdem der pH des Filtrates auf 6,5 bis 7,5 eingestellt worden ist. Als Lösungsmittel wird vorzugsweise Äthylacetat verwendet, der bevorzugte pH ist 7,0. Im Anschluß an die Extraktion wird der Lösungsmittelextrakt unter Vakuum eingedampft. Der Rückstand wird mit Petroläther behandelt Sp. (60° bis 80°C).

Das aus dem Myzel und dem Kulturfiltrat gewonnene, in Petroläther unlösliche Material wird kombiniert, gereinigt und mittels verschiedener chromatographischer Techniken wie "reversed phase"-RP-I8-Silikagel-Säulen-Chromatographie oder normaler Silikagel-Chromatographie oder präparativer Hochdruck-Flüssigchromatographie (HPLC) in Hexamycin A und Hexamycin B getrennt.

Die nachfolgenden Beispiele illustrieren die vorliegende Erfindung:

Beispiel I

Züchtung von Micromonospora species Y-82.200I2 (DSM 3777) für die fermentative Herstellung von Hexamycinen

Züchtung von Micromonospora species Y-82.200I2 auf CSPY-Medium mit folgender Zusammensetzung:

Casein   I,0 g
Maisstärke   I0,0 g
$K_2HPO_4$   0,5 g
Pepton   I,0 g
Hefeextrakt   I,0 g Wasser   I000 ml
pH   7,5

Das Medium wurde in Reagenzgläsern verteilt und 20 Minuten lang bei I2I°C sterilisiert. Die Reagenzröhrchen wurden zur Herstellung von Schrägagar in Schrägstellung abgekühlt. Die Schrägagar-Röhrchen wurden mit Micromonospora species Y-82.200I2 beimpft, die aus Bodenproben isoliert und 8 Tage lang bei 28°C inkubiert wurden, bis ein gutes Wachstum erkennbar war. Das Wachstum eines Schrägagars diente zum Inokulieren von fünf 500 ml-Erlenmeyer-Kolben, die jeweils 80 ml des folgenden Impfkulturmediums enthielten:
Zusammensetzung des Impfkulturmediums:

Rinderextrakt   3,0 g
Trypton   5,0 g
Sojabohnenmehl   5,0 g
Glucose   I,0 g
Lösliche Stärke   24,0 g
Hefeextrakt   5,0 g
$CaCO_3$   4,0 g
Wasser   I000 ml
pH   6,I

Das Impfkulturmedium wurde in Mengen zu 80 ml in 500 ml-Erlenmeyer-Kolben verteilt und 20 Minuten lang bei I2I°C sterilisiert. Die Kolben wurden abgekühlt, mit der Kultur beimpft und 72 Stunden lang bei 28°C auf einem Rotationsrüttler mit 3,75 cm Hub geschüttelt. Mit der gewachsenen Kultur wurde ein I5 Liter-Fermenter beimpft, der I0 Liter des beschriebenen Kulturmediums (I0 Vol.-%) für die Zubereitung der Zweitstufen-Impfkultur enthielt. Die Fermentierung erfolgte 24 Stunden lang bei 28°C (±I°C) unter Rühren bei 200 rpm und Belüftung bei 6 vvm. Die resultierende gut gewachsene Zweitstufen-Kultur wurde zum Beimpfen des folgenden Produktionsmediums verwendet:
Zusammensetzung des Produktionsmediums:

Lösliche Stärke   20,0 g
Glucose   I5,0 g
Soytone   3,0 g
Pepton   3,0 g
$CaCO_3$   4,0 g
$(NH_4)_2SO_4$   0,5 g
Maisquellwasser   2,0 g
NaCl   2,0 g

KJ    0,l g
CoCl₂    0,000l g
Spurenelementlösung    l,0 ml
Wasser    l000 ml
pH    6,l
Spurenelementlösung:

CuSO₄•5H₂O    7,0 g
FeSO₄•7H₂O    l,0 g
MnCl₂•4H₂O    8,0 g
ZnSO₄•7H₂O    2,0 g
Wasser    l000 ml

Dem Inhalt des Fermenters wurden 0,04 % $^R$Desmophen zugesetzt. 90 Liter des Produktionsmediums wurden in einen l50 Liter-Fermenter gegeben. Das Medium wurde durch indirekten und direkten Dampf 30 Minuten lang bei l2l°C sterilisiert. Der Fermenter wurde abgekühlt und mit der Zweitstufen-Impfkultur (l0 Vol.-%) beimpft. Die Fermentation erfolgte 72 Stunden lang bei 28°C unter Rühren bei 90 rpm. Die Belüftungsrate betrug 5 vvm. Nach Abbruch der Fermentation von 72 Stunden betrug der pH der Kulturbrühe 7,4 und das PCV 9 ml pro l00 ml.


Beispiel 2


Isolierung und Reinigung von Hextamycin A und Hextamycin B


Sämtliche Vorgänge während der Isolierung und Reinigung wurden in Abwesenheit von Licht ausgeführt.

Um das Filtrat (230 Liter) und das Mycel (7 kg) voneinander zu trennen, wurden die 250 Liter Kulturflüssigkeit zentrifugiert.

Der Mycel-Kuchen wurde dreimal mit Aceton extrahiert, wobei jeweils 80 Liter Lösungsmittel verwendet wurden. Das kombinierten Acetonextrakte wurden unter reduziertem Druck und bei einer Temperatur von 35°C konzentriert. Der zurückbleibende wäßrige Acetonextrakt wurde mit Wasser auf l2 Liter verdünnt und dann durch eine $^R$Diaion-HP-20-Säule (2 Liter) gegeben. Nach Eluieren der Säule mit Wasser (l0 Liter) und anschließend mit 50 % Methanol/Wasser (l0 Liter) wurde mit Methanol (l5 Liter) eluiert. Die Methanoleluate wurden unter reduziertem Druck zu einer Paste konzentriert und dann mit Petroläther behandelt, (Sp. 60° bis 80°C, l Liter). Das unlösliche gefärbte Material (3 g) enthielt das gewünschte Antibiotikum.

Das vorgenannte Roh-Antibiotikum wurde mit einer 30 Mikron-Octadecyldimethyl-silylierten Silikagel-Säule (l,7 Liter; 6 cm × 62 cm) chromatographiert. Die Säule wurde bei einer Fließrate von 55 ml/Min. zunächst mit einem Lösungsmittel-System (5 Liter) aus Methanol/Acetonitril /Wasser im Verhältnis 2 : 6 : 5 eluiert. Durch weiteres Eluieren mit Methanol/Acetonitril /0,2 % Natriumdihydrogenphosphat-Lösung im Verhältnis 2 : 6 : 3 erhielt man die Antibiotika-Mischung. Die Fraktionen (l0 Liter) mit dem darin enthaltenden Antibiotikum wurden unter reduziertem Druck bei 35°C bis zur Trockene konzentriert.

Das Pulver wurde mit Methanol trituriert. Der Methanollösliche Anteil wurde unter reduziertem Druck konzentriert und ergab eine feste Substanz (50 mg).

Hextamycin A und Hextamycin B wurden dann durch eine präparative Hochdruck-Flüssigchromatographie (HPLC) auf einer "reversed phase"-Säule mit Oktadecyldimethylsilyliertem Silikagel mit Methanol/Acetonitril /0,2 % Natriumhydrogenphosphat im Verhältnis 4 : 3 : 3 als Eluent voneinander getrennt. Die mit zwei getrennten Peaks eluierten beiden Antibiotika wurden getrennt gesammelt und unter reduziertem Druck bei 35°C jeweils bis zur Trockene konzentriert. Die Pulver wurden jeweils mit Methanol trituriert, der Methanol-lösliche Anteil wurde konzentriert, erneut in Chloroform gelöst und durch Zusatz von Petroläther (Sp. 60° bis 80°C) ausgefällt. Durch dieses Verfahren wurden aus 50 mg Gemisch 5 mg Hextamycin A und 3 mg Hextamycin B als farblose feste Substanzen erhalten.


Eigenschaften von Hextamycin A


Physikochemische Eigenschaften: Farbe:    farblos
Löslichkeit:    in Wasser unlöslich, löslich in Methanol Aceton, Dimethylformamid
UVmax:    keine selektive Absorption.

Eigenschaften von Hextamycin B

Physikochemische Eigenschaften: Farbe:    farblos
Löslichkeit:    unlöslich in Wasser, löslich in Methanol Aceton, Dimethylformamid
UVmax:    keine selektive Absorption.

Die antibiotische Wirksamkeit der beider erfindungsgemäßen Verbindungen ist in den folgenden Tabellen I und 2 aufgeführt.

Tabelle 1

Minimale Hemmkonzentration vom Hextamycin A (MHK in µg/ml)

| Nr. | Test-Mikroorganismus | MHK (µg/ml) |
|---|---|---|
| 1. | S. aureus 209 P | 0.00012 |
| 2. | S. aureus 20240 | 0.0005 |
| 3. | S. aureus E-88 (Meth$^{R+}$) | 0.00025 |
| 4. | S. aureus 3066 (" ) | 0.00025 |
| 5. | S. aureus 20424 (" ) | 0.002 – 0.0625 |
| 6. | S. aureus 20666 (" ) | 0.00025 |
| 7. | Str. faecalis | 0.004 |
| 8. | Str. D. Eder | 0.002 |
| 9. | B. megaterium | 0.008 |
| 10. | Micrococcus luteus | 0.001 |
| 11. | Ess 2231 | 0.5 |
| 12. | E. coli 9632 | 1.0 |
| 13. | E. coli 20683 | 1.0 |
| 14. | Pr. vulgaris | 0.25 |
| 15. | Pr. rettgeri 21207 | 0.25 |
| 16. | Ent. cloacae P-99 | 1.0 |
| 17. | Alcaligenes faecalis | 0.25 |
| 18. | Citrobacter sp. | 0.5 |
| 19. | Serr. mar. 20460 | 0.25 |
| 20. | Sal. typhimurium | 1.0 |
| 21. | Kl. pneu. 1976 E | 1.0 |
| 22. | Ps. aeruginosa | 0.5 |
| 23. | Ps. a. M-35 | 0.25 |
| 24. | Ps. a. 1592 E | 0.5 |
| 25. | Ps. a. 20897 | 0.5 |
| 26. | Ps. a. 20653 | 0.5 |
| 27. | Ps. a. 80 | 0.5 |
| 28. | Ps. a. J-61 | 0.25 |
| 29. | Ps. a. J-62 | 0.25 |
| 30. | Ps. a. NCTC 10701 | 0.5 |

Tabelle 2

Minimale Hemmkonzentration vom Hextamycin B (MHK in µg/ml)

| Nr. | Test-Mikroorganismus | MHK (µg/ml) |
|-----|---------------------|-------------|
| 1. | S. aureus 209 P | 0.00003 |
| 2. | S. aureus 20240 | 0.00012 |
| 3. | S. aureus E-88 (Meth$^{R+}$) | 0.00006 |
| 4. | S. aureus 3066 (" ) | 0.00006 |
| 5. | S. aureus 20424 (" ) | 0.016 |
| 6. | S. aureus 20666 (" ) | 0.00006 |
| 7. | Str. faecalis | 0.00025 |
| 8. | Str. D. Eder | 0.00025 |
| 9. | B. megaterium | 0.001 |
| 10. | Micrococcus luteus | 0.00025 |
| 11. | Ess 2231 | 0.0625 |
| 12. | E. coli 9632 | 0.25 |
| 13. | E. coli 20683 | 0.25 |
| 14. | Pr. vulgaris | 0.0625 |
| 15. | Pr. rettgeri 21207 | 0.0625 |
| 16. | Ent. cloacae P-99 | 0.25 |
| 17. | Alcaligenes faecalis | 0.0625 |
| 18. | Citrobacter sp. | 0.25 |
| 19. | Serr. mar. 20460 | 0.0625 |
| 20. | Sal. typhimurium | 0.25 |
| 21. | Kl. pneu. 1976 E | 0.25 |
| 22. | Ps. aeruginosa | 0.0625 |
| 23. | Ps. a. M-35 | 0.0625 |
| 24. | Ps. a. 1592 E | 0.0625 |
| 25. | Ps. a. 20897 | 0.0625 |
| 26. | Ps. a. 20653 | 0.625 |
| 27. | Ps. a. 80 | 0.0625 |
| 28. | Ps. a. J-61 | 0.0312 |
| 29. | Ps. a. J-62 | 0.0625 |
| 30. | Ps. a. NCrC 10701 | 0.125 |

Man kann aus diesen Daten ersehen, daß Hextamycin A und Hextamycin B sich von anderen, aus der Literatur bekannten Antibiotika unterscheiden, und daß die antibiotische Wirksamkeit der erfindungsgemäßen Verbindungen außergewöhnlich hoch ist.

Die erfindungsgemäßen Verbindungen zeigen auch eine hohe zytotoxische Wirkung gegen Leukämie- und Karzinomzellen wie aus den folgenden experimentellen Daten hervorgeht(getestet wurde ein Gemisch von 80 % Hextamycin A und 20 % Hextamycin B).

A. Zytotoxizität gegen L 1210 Leukämiezellen

Experimentelles Vorgehen:

Der Test wurde entsprechend der von Hamburger und Salmon beschriebenen Methodik mit den unten beschriebenen Modifikationen durchgeführt.

Konditioniertes Medium wurde durch McCoy 5A Medium ersetzt. Als Folge der hohen Klonierungsrate der L1210 Leukämiezellen in Soft Agar wurde die Anzahl an Tumorzellen pro Platte auf $5 \times 10^2$ reduziert.

Die Zellen wurden mit unterschiedlichen Konzentrationen der Testsubstanz für 1 Stunde bei 37°C inkubiert. Anschließend wurden die Zellen zweimal mit McCoy 5A Medium gewaschen und anschließend in einem Zwei-Schichten-Agar System als obere Schicht entsprechend der Methode von Hamburger und Salmon ausplatiert.

Zusätzliche Parallelexperimente wurden unter Verwendung einer kontinuierlichen Inkubationszeit durchgeführt, wobei unterschiedliche Konzentrationen der Testsubstanz der oberen Agarschicht vor Ausplatieren der Zellen zugemischt wurden.

Die Platten wurden in einem Brutschrank mit 5 % $CO_2$, 20 % $O_2$ und 95 % relativer Luftfeuchte für 5 - 7 Tage inkubiert. Nach dieser Zeit wurden Kolonien mit einen Durchmesser > 60 $\mu$m mit Hilfe eines Invertoskopes gezählt.

Die Ergebnisse wurden angegeben als prozentualer Anteil der Kolonien in behandelten versus unbehandelten Gruppen. Der Variationskoeffizient, bei wiederholten Experimenten war kleiner als 15 %.

Resultate:

Aus der Dosis-Wirkungskurve wurde die $IC_{50}$ für die kontinuierliche und einstündige Inkubation bestimmt (Tab. 3).

## Tabelle 3

$$IC_{50} \ (\mu g/ml)$$

| Stamm-Zell-Assay | |
|---|---|
| Kontinuierl. Inkubation | 1 Std. Inkubation |
| $6,7 \times 19^{-6}$ | $1,9 \times 10^{-5}$ |

B. In vivo-Wirksamkeit gegen L 1210-Leukämie, B 16 Melanom und Lewis Lung Adenocarcinom

Methodik:

L1210: Tumorzellpräparation:
Aszites Flüssigkeit wird 7 Tage nach Implantation unter sterilen Bedingungen aus DBA2 Mäusen entnommen (weiblich, 18 bis 20 g). Die Aszites Flüssigkeit wird dreimal mit PBS gewaschen, gezählt und anschließend in PBS verdünnt bis zu einer Endkonzentration von $10^6$ Zellen pro 0,2 ml.
Tumortransfer:

$10^6$ Zellen in 0,2 ml PBS werden DBA2 Mäusen (weiblich , 18 bis 20 g) intraperitoneal appliziert. Dieser Transfer wird einmal wöchentlich wiederholt.

Transfer zur antitumoralen Testung:
$10^5$ Zellen in 0,2 ml PBS werden BDFI Mäusen (weiblich, 18 bis 20 g) intraperitoneal appliziert. 6 Tiere pro Gruppe werden für jede Substanzkonzentration sowie für die Kontrolle eingesetzt.
B16 Melanom und Lewis Lung Adenokarzinom:

Tumoraufarbeitung:
Der subcutan wachsende solide Tumor wird unter sterilen Bedingungen 14 bis 18 Tage nach Implantation in C57Bl/6 Mäusen (weiblich, 18 bis 20 g) entnommen. Der Tumor wird kleingeschnitten und mit Kollagenase (0,05 %) bei 37°C für eine Stunde behandelt. Die entstehende Einzelzellsuspension wird dreimal mit PBS gewaschen, gezählt und schließlich auf eine Endkonzentration von $10^6$ Zellen/0,2 ml verdünnt.

Tumortransfer:
$10^6$ Zellen in 0,2 ml PBS werden C57Bl/6 Mäusen (weiblich , 18 bis 20 g) ungefähr alle 14 Tage subcutan injiziert, um so die Zellinie in Passage zu erhalten.

Transfer zur antitumoralen Testung:
$10^6$ Zellen in 0,2 ml PBS werden BDFI Mäusen (weiblich, 18 bis 20 g) intraperitoneal appliziert. 6 Tiere pro Gruppe werden für jede Substanzkonzentration und für die Kontrolle eingesetzt.

Ermittlung des antitumoralen Effektes:

a) Die Tiere werden an Tag 1 und Tag 5 nach Tumorzellimplantation gewogen. Gewichtsverlust von mehr als 20 % an Tag 5 wird als Indikator eines toxischen Substanzeffektes verwendet.

b) Am Ende des Experimentes (Tod aller Tiere oder Erreichen von Tag 60) wird die mediane Überlebenszeit der Behandlungsgruppen bestimmt, sofern diese > 65 % überlebende tiere am Tag 5 enthielten. Die Ermittlung der medianen Überlebenszeit erfolgt entsprechend der Formel:

$$MST = \frac{(X + Y)}{2}$$

In dieser Formel bedeutet X den frühesten Tag, an dem die Zahl der überlebenden Tiere < N/2 und Y den frühesten Tag, an dem die Zahl der überlebenden Tiere < (N/2)-1 ist. In dem Fall, daß N eine ungerade Zahl ist, entspricht die mediane Überlebenszeit dem Zeitpunkt X.

Die mediane Überlebenszeit wird ausschließlich für im Verlauf des Experimentes sterbende Tiere bestimmt. Geheilte Tiere (LTS) werden von der Bestimmung der medianen Überlebenszeit ausgeschlossen und getrennt aufgeführt.

Aus der medianen Überlebenszeit der behandelten ($MST_T$) und Kontrollgruppen ($MST_C$) wird der antitumorale Effekt (T/C) entsprechend der Formel

$$T/C \% = \frac{MST_T}{MST_C} \times 100$$

bestimmt. T/C-Werte von mehr als 125 % werden als Indikator einer signifikanten antitumoralen Wirksamkeit der Testverbindungen angesehen. Die Dosis, die den größten antitumoralen Effekt bewirkt (optimale Dosierung) sowie jeweils eine Dosierung oberhalb und unterhalb dieser Dosierungsstufe werden in Tabelle 4 zusammengefaßt. Lebende Tiere am Tag 60 werden als geheilt betrachtet (LTS).

Behandlungsplan:

3 ˣ i.p./i.p. (Q3D), beginnend am Tag I nach Tumorimplantation.

Tabelle 4

| L 1210 | | | |
|---|---|---|---|
| T/C | Optimale Dosis (µg/kg/inj) | LTS | Toxische Todesfälle |
| 117 | 3,0 | 2/6 | 0/6 |
| 139 | 5,3 | 1/6 | 0/6 |
| 148 | 9,4 | - | 2/6 |

Die Verbindungen und ihre Säureadditionssalze können als Medikamente zur Behandlung von Tumoren verwendet werden. Die Verbindungen können auf verschiedene Weise in Abhängigkeit von der Dosierungsform verabreicht werden.

Normalerweise werden die Verbindungen oder ihre Säureadditionssalze, z. B. mit D-Gluconsäure, mit pharmazeutisch üblichen Trägerstoffen oder Verdünnungsmitteln vermischt verabreicht. So können sie z. B. einzeln oder in Mischung zusammen mit Trägerstoffen wie Maltose oder Lactose oder als nicht-toxische Komplexe, z. B. als Desoxyribonucleinsäure-Komplex, verabreicht werden.

Eine typische Verabreichungsart ist die Injektion einer Lösung der erfindungsgemäßen Verbindungen in destilliertem Wasser oder in physiologischer Kochsalzlösung. Die Lösungen können intraperitoneal, intravenös oder intraarteriell injiziert werden.

Tagesdosis und Einheitsdosis können aus Tierversuchen und auch aus in vitro-Tests in der Weise festgesetzt werden, daß die Gesamtdosis, die kontinuierlich oder in Abständen verabreicht wird, einen vorher festgelegten Bereich nicht überschreitet. So beträgt die Gesamtdosis für einen Behandlungszyklus etwa 0,5-5 mg/kg Körpergewicht. Diese Dosis kann in entsprechenden Bruchteilen über einen Zeitraum von 7 Tagen verabreicht werden. Es ist jedoch klar, daß konkrete Dosen für die Behandlung von Mensch oder Tier individuell festgelegt werden können in Abhängigkeit von der jeweiligen Situation des Patienten, z. B. Alter, Körpergewicht, Geschlecht, Empfindlichkeit, Nahrung, Zeitpunkt der Verabreichung, weiteren verabreichten Medikamenten, körperlichem Zustand der Patienten und Schwere der Erkrankung.

**Ansprüche**

1. Antibiotisch wirksame Verbindungen, Hextamycin A und Hextamycin B, dadurch gekennzeichnet, daß sie hergestellt werden durch Kultivieren des Mikroorganismus-Stammes Micromonospora species Y-82.20012 (DSM 3777), seiner Varianten oder Mutanten, unter aeroben Bedingungen in einem üblichen Nährmedium , gegebenenfalls in Gegenwart von Inhibitoren von Antibiotika-abbaubaren Enzymen, Isolierung der Verbindungen aus der Kulturbrühe und dem Myzel durch Extraktion mit mit Wasser nicht mischbaren Lösungsmitteln und anschließende Trennung und Reinigung der Verbindungen aus dem Lösungsmittel-Extrakt durch chromatographische Methoden.

2. Verbindungen gemäß Anspruch 1, dadurch gekennzeichnet, daß sie hergestellt werden durch Kultivieren des Mikroorganismus-Stammes bei Temperaturen zwischen 28 und 32°C, einem pH von 6,0 bis 8,0 und einer Kultivierungszeit von 72 bis 96 Stunden.

3. Verbindungen gemäß Anspruch 1, dadurch gekennzeichnet, daß sie aus dem Lösungsmittelextrakt durch Silikagel-Chromatographie, "reserved phase"-Silikagel-Säulenchromatographie oder präparativer HPLC isoliert werden.

4. Arzneimittel, gekennzeichnet durch einen Gehalt an Hextamycin A und/oder B gemäß Anspruch 1 und einem festen, flüssigen oder halbflüssigen Träger-oder Hilfsstoff.

5. Verwendung von Hextamycin A oder Hextamycin B oder einem Gemisch dieser Verbindungen zur Herstellung von Arzneimitteln mit antibiotischer und cytostatischer Wirkung.

6. Micromonospora species Y-82.20012 (DSM 3777).

10

Patentansprüche für folgende Vertragsstaaten : AT; ES

1. Verfahren zur Herstellung antibiotisch wirksamer Verbindungen Hextamycin A und Hextamycin B, dadurch gekennzeichnet, daß man den Mikroorganismus-Stamm Micromonospora species Y-82.20012 (DSM 3777) oder einen seiner Varianten oder Mutanten, unter aeroben Bedingungen in einem üblichen Nährmedium , gegebenenfalls in Gegenwart von Inhibitoren von Antibiotika-abbaubaren Enzymen kultiviert, die Verbindungen aus der Kulturbrühe und dem Myzel durch Extraktion mit mit Wasser nicht mischbaren Lösungsmitteln und anschließend aus dem Lösungsmittel-Extrakt mittels chromatographischer Methoden isoliert.

2. Verfahren gemäß Anspruch 1, dadurch gekennzeichnet, daß der Mikroorganismus-Stamm bei Temperaturen zwischen 28 und 32°C, einem pH von 6,0 bis 8,0 und 72 bis 96 Stunden lang kultiviert wird.

3. Verfahren gemäß Anspruch 1, dadurch gekennzeichnet, daß man die Verbindungen aus dem Lösungsmittelextrakt durch Silikagel-Chromatographie, "reserved phase"-Silikagel-Säulenchromatographie oder präparative HPLC isoliert.

4. Arzneimittel, gekennzeichnet durch einen Gehalt an Hextamycin A und/oder B gemäß Anspruch 1 und einem festen, flüssigen oder halbflüssigen Träger-oder Hilfsstoff.

5. Verwendung von Hextamycin A oder Hextamycin B oder einem Gemisch dieser Verbindungen zur Herstellung von Arzneimitteln mit antibiotischer und cytostatischer Wirkung.

6. Micromonospora species Y-82.20012 (DSM 3777).